# EUROPEAN PATENT SPECIFICATION

(11) **EP 4 244 622 B1**
(45) Date of publication and mention of the grant of the patent: **13.08.2025**
(21) Application number: 21839630.7
(22) Date of filing: 21.12.2021
(51) Int. Cl.: G01N 33/50, G01N 33/569

(54) **METHOD FOR DIAGNOSING AND MONITORING SEPSIS**
VERFAHREN ZUR DIAGNOSE UND ÜBERWACHUNG VON SEPSIS
PROCÉDÉ DE DIAGNOSTIC ET DE SURVEILLANCE D'UNE SEPTICÉMIE

(30) Priority: 22.12.2020 GB 202020402; 23.12.2020 GB 202020503; 24.05.2021 US 202163192232 P
(43) Date of publication of application: 20.09.2023
(73) Proprietor: Seroxo Limited, Richmond, Surrey TW10 6QA (GB)
(72) Inventor: SARPHIE, David, Richmond ,Surrey TW10 6QA (GB); MIAN, Rubina, Harborne ,Birmingham B17 0RE (GB)
(74) Representative: HGF
(86) International application number: PCT/GB2021/053381
(87) International publication number: WO 2022/136850

(56) References cited:
- WO-A1-2017/122203
- WO-A1-2018/060741
- WO-A1-2020/239622
- WO-A1-2020/263571
- WO-A1-94/29728
- DE-A1- 102004 031 560
- PASCUAL C ET AL: "A controlled study of leukocyte activation in septic patients", INTENSIVE CARE MEDICINE, SPRINGER BERLIN HEIDELBERG, BERLIN/HEIDELBERG, vol. 23, no. 7, 1 July 1997 (1997-07-01), pages 743 - 748, XP037084043, ISSN: 0342-4642, DOI: 10.1007/S001340050403
- JUNDI BAKR ET AL: "Leukocyte function assessed via serial microlitre sampling of peripheral blood from sepsis patients correlates with disease severity", NATURE BIOMEDICAL ENGINEERING, NATURE PUBLISHING GROUP UK, LONDON, vol. 3, no. 12, 11 November 2019 (2019-11-11), pages 961 - 973, XP037075509, DOI: 10.1038/S41551-019-0473-5
- CHRISTIAN BIME ET AL: "Reactive oxygen species–associated molecular signature predicts survival in patients with sepsis", PULMONARY CIRCULATION, vol. 6, no. 2, 7 April 2016 (2016-04-07), US, pages 196 - 201, XP055371998, ISSN: 2045-8932, DOI: 10.1086/685547
- MAGRISSO M ET AL: "Fiber-optic biosensor to assess circulating phagocyte activity by chemiluminescence", BIOSENSORS AND BIOELECTRONICS, ELSEVIER SCIENCE LTD, UK, AMSTERDAM , NL, vol. 21, no. 7, 15 January 2006 (2006-01-15), pages 1210 - 1218, XP024961403, ISSN: 0956-5663, [retrieved on 20060115], DOI: 10.1016/J.BIOS.2005.05.006

## Description

### Field of the invention

The present invention relates to rapid *in vitro* methods for diagnosing sepsis and monitoring sepsis progression and treatment in individuals. More particularly, methods are provided which rely on determination of the responsiveness of leukocytes (predominately neutrophils) to exhibit challenge-induced superoxide anion production, i.e. produce a "respiratory burst" in response to *in vitro* activation. While such activation of leukocytes is known and is often referred to as leukocyte coping capacity (LCC), the invention provides new application in the field of sepsis diagnosis whereby simple and quick indicative diagnosis of sepsis can be achieved using whole blood samples without any need for fractionation. The invention has been shown to differentiate sepsis patients from healthy volunteers with highly significant difference in measured superoxide anion (also known as "reactive oxygen species") production. It is therefore envisaged that the invention has application generally in providing quick indicative diagnosis of sepsis, including septic shock.

Sepsis is commonly defined as life-threatening organ dysfunction caused by a dysregulated host response to infection (The International Sepsis Task Force, Singer et al. 2016). Currently clinical evaluation of onset or occurrence of sepsis requires assessment of a variety of clinical criteria while prompt treatment is desirable. The present invention permits quick indicative diagnosis where at best infection may be suspected but awaits confirmation. Whole blood samples from septic shock patients exhibit LCC scores which are far higher than for whole blood samples from healthy volunteers, the difference being of such surprising magnitude that LCC scores are seen as providing an immediate means of differentiating sepsis patients.

Such methodology may be applied in a number of areas of clinical practice including: i.) in hospital intensive care units (ICUs) to provide a rapid method of sepsis screening and monitoring of treatments, ii.) in Accident & Emergency (A&E) units and iii.) in ambulances where rapid screening for sepsis is critical, and iv.) in general practice (GP) surgeries as a potential method of diagnosing septicaemia, a bacterial infection of the blood, which can lead to sepsis.

It may provide quick and convenient guidance for moderating therapeutic interventions in sepsis, for example the dosages and the time intervals between doses, changes of drug or dosage or change of form of treatment.

### Background to the invention

Sepsis is one of the most common causes of death among hospitalized patients in the intensive care unit (ICU). It is particularly difficult to diagnose in this setting because of the multiple comorbidities and underlying diseases that these patients present (Novosad et al. 2016; Vincent et al. 2009.)

Sepsis is a medical emergency that describes the body's systemic immunological response to an infectious process that can lead to end-stage organ dysfunction and death (Gyawali et al. 2019). Sepsis remains one of the major causes of morbidity and mortality in critically ill patients (Kaukonen et al. 2014).

Early detection of sepsis is critical to starting, maintaining and monitoring effective treatment. Kumar et al. (2006) demonstrated a clear association between a delay in effective antibiotic initiation after the onset of hypotension and in-hospital mortality. Each hour of delay was associated with a decrease in survival of approximately 7% on average. The administration of antibiotics within the first hour of recognition of sepsis or septic shock resulted in survival rates of up to 80%.

Chemical inducers such as phorbol myristate acetate (PMA) are known for activating neutrophils in peripheral blood samples whereby retained capacity for reactive oxygen species production by leucocytes (predominately neutrophils) can be quantified (Hu et al. Cell Signal (1999) 11, 335-360). Such use with whole blood samples with measurement of reactive oxygen species production by chemiluminescence detection so as to obtain an LCC score forms the basis of a test commercialised by Oxford MediStress Limited to quantify physiological stress in humans and animals; see European Patent no. 1558929 and related patents deriving from published International Application WO2004/042395. However, the data reported herein provides for the first time evidence for very different utility of the same type of blood test in determining onset and occurrence of sepsis or septic shock and monitoring treatment thereof; see Example 2.

Thus there is now enabled a point-of-care (POC) *in vitro* diagnostic (IVD) that uses the body's own cells (leukocytes) in whole blood to act as bio-indicators of sepsis (referred to as the Leukocyte ImmunoTest^{™} or LIT^{™}) . Leukocytes respond to pro- and anti-inflammatory mediators and pick up signals from immunological and non-immunological pathways, cardiovascular, autonomic, neurological, hormonal, metabolic and clotting. One of the activities of neutrophils *in vivo* is the production of reactive oxygen species (ROS). This is an essential step in the killing of bacteria. All these systems are affected by sepsis (Segal et al. 2005).

Published International Application WO94/29728 discloses an *in vitro* method for diagnosing sepsis requiring use of antibody specific for an antigen indicative of bacterial infection to activate ROS production. Pascual et al. (1997) Intensive Care Med. 23, 743-748 entitled 'A controlled study of leucocyte activation in septic patients discloses in part chemiluminescent detection of PMA-induced superoxide production in whole blood samples from septic patients requiring using an arterial blood sample of 50µl collected into a conventional EDTA- K tube. Incubation with the chemiluminescent agent is taught for 65 minutes at 37°C with measurement of the maximal light emitted during this period. However, there is now taught a far more rapid test enabling detection of sepsis in 10 minutes applicable to a finger-prick whole blood sample of 10-20µl.

Current techniques for diagnosing sepsis often use clinical observations of a patient's vital signs (in particular, temperature, heart rate, respiratory rate and blood pressure) as well as behaviour (signs of confusion). See Table 1 below which sets out the UK National Institute of Clinical Excellence (NICE) guidelines for risk stratification of sepsis.

The International Sepsis Task Force [Singer et al. (2016) JAMA, The Third International Consensus Definitions for Sepsis and Septic Shock (Sepsis-3)] has provided an alternative screening tool for sepsis in adult patients with suspected infection who are likely to have poor outcomes which recognises the significance of need for sequential organ failure assessment (SOFA). Termed 'quick SOFA' (qSOFA), this provides definition of sepsis using a qSOFA score to identify adult patients with suspected infection and liable to have poor outcome : respiratory rate ≥ 22/min, altered mentation (e.g. Glasgow Coma scale <15) and systolic blood pressure ≤100mm Hg.

Septic shock (sometimes termed Sepsis-3) is a category of sepsis in which particularly profound circulatory, cellular and metabolic abnormalities are associated with a substantially greater risk of mortality. It is recognised by the combination of hypotension (vassopressor requirement to maintain a mean arterial pressure of 65 mm Hg or greater) and increased serum lactate >2 mM [see again Singer et al.(2016) The Third International Consensus Definitions for Sepsis and Septic Shock (Sepsis-3)].

The diagnosis of sepsis in critically ill patients is nevertheless challenging because it can be complicated by the presence of inflammation as a result of other underlying disease processes and prior use of antibiotics making cultures negative. In addition, culture-dependent diagnosis of infection is slow.

### Summary of the invention

As indicated above, it has now been surprisingly found that a simple whole blood test can be used to gain a rapid quantitative measure of immune status as an indicative assessment of the likely presence of sepsis or septic shock in a patient by relying on determination of challenge-induced superoxide production by leukocytes; see Example 2. Such a test protocol has the advantage that it provides a rapid result (in ten minutes) and may be conducted in the hospital ward or ICU (near the patient) to assess neutrophil function and provide warning of changes in immune status associated with development of sepsis or septic shock.

The method of the invention as now disclosed is not only rapid, but significantly uses whole blood without centrifugations so the sensitivity of the cells remains intact and the cells maintain signalling capacity with each other, red blood cells, cytokines in the blood and plasma.

Of especial interest is that such a test may be applied to blood samples from patients suffering from sepsis whereby LCC score may be correlated with disease status. Such a score is hereinafter referred to in the clinical context as an LIT score. For example, importantly it may provide a rapid indicative diagnosis of the onset or occurrence of high risk sepsis status (according to the criteria for example of the UK NICE guidelines for risk stratification of suspected sepsis, qSOFA score as defined above, or alternative criteria for equivalent assignment of sepsis status) or septic shock, or enable determination of such. Such a ranking or stratification method is considered to be useful for monitoring disease progression or treatment.

Whilst the invention relies on assessing the capacity of the total leukocyte component of whole blood to produce superoxide in response to appropriate *in vitro* chemical challenge, (commonly referred to as the leukocyte coping capacity (LCC) score), since this will largely be determined by neutrophil capability for such external stimulation, reference may alternatively be made to the individual's neutrophil functionality level.

In its broadest aspect, the present invention thus provides a method of providing an indicative diagnosis of the onset or occurrence of sepsis or septic shock, or determining the onset or occurrence of sepsis or septic shock, in a subject known to have an infection with an agent capable of giving rise to sepsis or suspected of having such an infection, which comprises:
(a) directly contacting a test whole blood sample of 10-20µl obtained from said subject with an inducer capable of stimulating superoxide production in neutrophils, said inducer being phorbol myristate acetate (PMA) and said contacting being at a temperature of 37-37.5° C under conditions suitable for such stimulation;
(b) determining the increase of superoxide production above basal in said test sample after a pre-determined time period of 10 minutes to obtain a first test result, wherein superoxide production is detected by chemiluminescence detection using luminol and
(c) comparing said first test result with a second comparator result which is a threshold obtained by applying steps (a) and (b) to whole blood samples of healthy individuals and utilising the increase of chemiluminescence of said first test result above the increase of chemiluminescence of said second comparator result,
whereby the onset, presence or absence of sepsis or septic shock in said subject is indicated or determined

In one aspect of the invention, there is provided a method of ranking or scoring a sepsis patient on the basis of sepsis progression or presence of septic shock, which comprises:
(a) directly contacting a test whole blood sample of 10-20µl obtained from said patient with an inducer capable of stimulating superoxide production in neutrophils, said inducer being phorbol myristate acetate (PMA) and said contacting being at a temperature of 37-37.5°C under conditions suitable for such stimulation;
(b) determining the increase of superoxide production above basal in said test sample after a pre-determined time period of 10 minutes to obtain a first test result, wherein superoxide production is detected by chemiluminescence detection using luminol; and
(c) comparing said first test result with one or more second comparator results each of which is a pre-determined threshold which correlates with one or more criteria for sepsis progression or septic shock so as to rank or score sepsis progression or presence of septic shock in the subject,
wherein the one or more second comparator results are derived by applying steps (a) and (b) to whole blood samples of healthy individuals and utilising the increase of chemiluminescence of said first test result above the increase of chemiluminescence of said second comparator result.

In one aspect of the invention, there is provided a method for determining the
effectiveness of the treatment of sepsis or septic shock in a subject, said method comprising, following or during sepsis or septic shock treatment,
(a) directly contacting a test whole blood sample of 10-20µl obtained from said subject with an inducer capable of stimulating superoxide production in neutrophils, said inducer being phorbol myristate acetate (PMA) and said contacting being at a temperature of 37-37.5°C under conditions suitable for such stimulation;
(b) determining the increase of superoxide production above basal in said test sample after a pre-determined time period of 10 minutes to obtain a first test result, wherein superoxide production is detected by chemiluminescence detection using luminol ;and
(c) comparing said first test result with a second comparator result which is a threshold derived by applying steps (a) and (b) to whole blood samples of healthy individuals and utilising the increase of chemiluminescence of said first test result above the increase of chemiluminescence of said second comparator result; and
   wherein the increase in chemiluminescence above basal in said test sample is compared with the increase in chemiluminescence above basal in a second comparator sample which has been taken from the subject at an earlier time point at the start or during treatment,
   whereby reduction in induced chemiluminescence in the test sample compared with in said second comparator sample is indicative of depressive effect of the treatment on neutrophil functionality.

Generally, the subject will have a known infection with an agent capable of giving rise to sepsis such as a bacterial or viral infection, or at least be suspected of having such an infection. Where a method of the invention provides at least an indicative diagnosis of the development or presence of sepsis or septic shock, this may be used to prompt appropriate medical intervention with the aim of preventing sepsis or septic shock or increase of severity of sepsis or septic shock. As noted above, the method of the invention is especially useful for example as a rapid diagnostic test for onset or occurrence of high risk sepsis or septic shock; the increase of superoxide production determined in blood samples of such individuals is of such an order of magnitude compared to that seen in blood samples of healthy individuals that onset or occurrence of high risk sepsis or septic shock is directly indicated or diagnosed.

### Brief description of the figure

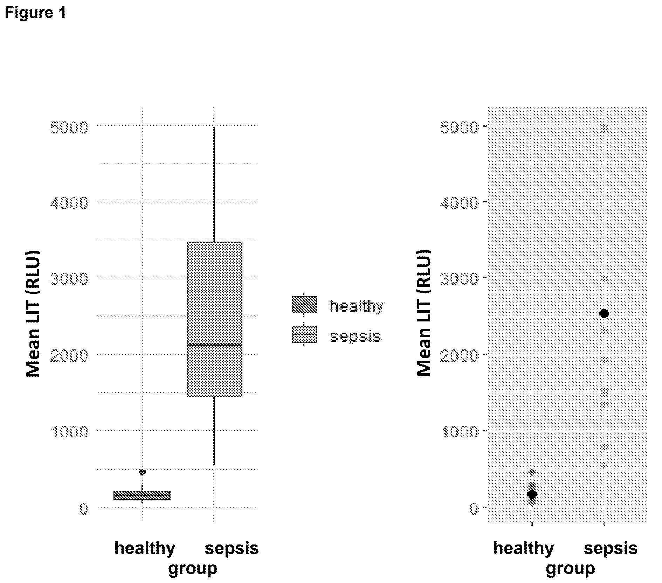

Figure 1 illustrates use of LIT scores from a controlled clinical trial wherein LIT scores from patients clinically confirmed to have sepsis were compared with those from a healthy control group as discussed further in the exemplification. The results used are means obtained with triplicate whole blood samples. The boxplot shows the median and interquartile range of the scores for both groups. The dot plot shows individual subject values and, as darker dots, the mean for both groups.

### Detailed description

In a method of the invention where superoxide production is determined after a short period of PMA-challenge by conventional chemiluminescence measurement employing luminol and e.g. a portable luminometer, the basal chemiluminescence invariably will be so low in samples as not to require consideration. Thus in these circumstances, total measured relative light units (RLUs) may be equated with induced superoxide production and as directly proportional to neutrophil functionality. This has been established to apply for example when using freeze-dried PMA/ luminol reagent as supplied by Oxford MediStress for LCC scoring of whole blood samples and incubating the sample for 10 minutes at 37.5°C in accordance with the standard protocol for use of that reagent. In other words, "determining the increase of superoxide production above basal" in the test sample may be equated with simple one step quantification of superoxide present at the end of the neutrophil stimulation period (10 minutes after addition of the inducer).

Comparison step (c) may enable sepsis or septic shock to be directly diagnosed where superoxide production above basal determined in step (b) for a test sample is higher than a threshold obtained by applying steps (a) and (b) to equivalent samples from healthy individuals, but it will be appreciated that the second comparator result may be any alternative comparator result or threshold which enables either indicative diagnosis of sepsis or septic shock or monitoring of progression or treatment.

Thus, determining the presence of sepsis or septic shock may preferably extend to providing a prognostic indication of likely severity of disease. Thus the second comparator result may be a pre-determined threshold recognised to indicate the presence or absence of sepsis and/or septic shock.

A method of the invention may be applied following or during sepsis or septic shock treatment to determine the effectiveness of the treatment or monitor the treatment. In this case, in step (c) the increase in superoxide production above basal in the test sample may be compared with the increase in superoxide production above basal in a second comparator sample which has been taken from the subject at an earlier time point at the start or during treatment. Samples taken from individuals for the purpose of the present invention will generally be taken such that differential effects of psychological stress between the test sample and any comparator sample are minimized.

Thus in an embodiment, the invention provides a method for assessing effectiveness of treatment in a subject of sepsis or septic shock which comprises the steps of:
(i) directly contacting a whole blood sample of 10-20µl obtained from the patient after a period of treatment with an inducer of superoxide production in neutrophils, said inducer being PMA and said contacting being at a temperature of 37-37.5 °C under conditions suitable for stimulating superoxide production in neutrophils;
(ii) determining the increase of superoxide production above basal in said test sample after a time period of 10 minutes by chemiluminescence detection using luminol to obtain a first result, and
(iii) comparing said first result with a second control result derived from a whole blood sample taken from the patient at an earlier time point prior to or during treatment, whereby reduction in induced increase in superoxide production in said test ample compared with the control result is indicative of depressive effect of the treatment on neutrophil functionality level.

Steps (i) to (iii) may be repeated thereby providing a highly convenient means for long-term ("longitudinal") monitoring of the effect of sepsis treatment on the patient's physiological status. Such methodology is envisaged as a convenient means for example for aiding decision-making by clinicians in applying treatment, for example dosages, the time interval between dosages which will be founded on clinically relevant cell function rather than just counting cells or looking at structure.

In a further embodiment of the invention, a method of the invention with steps (a) to (c) as defined above may be applied to a plurality of samples from patients expected to have various degrees of sepsis and/or septic shock, and comparison of results of quantification of induced superoxide production (LCC score) used to assign each sample to a rank by correlation with one or more criteria indicative of disease progression. Such stratification will provide pre-determined thresholds which may be employed as a second comparator result in applying a method of the invention to further samples from patients suspected or known to have sepsis or septic shock to determine disease progression, monitor effectiveness of treatment, or provide a prognostic indicator.

Thus the invention extends to a method of ranking or scoring a sepsis patient on the basis of sepsis progression or presence of septic shock which comprises applying steps (a) to (c) of a method of the invention as above to a whole blood sample from the patient wherein in step (c) said first test result is compared with one or more second comparator results each of which is a pre-determined threshold which correlates with one or more criteria for sepsis progression or septic shock. For example, said first test result may be compared with a pre-determined threshold for onset or occurrence of septic shock. The test result of the patient may be compared with one or more thresholds indicative of sepsis severity based on known scoring for sepsis progression, e.g. progression to moderate to high risk and/ or high risk on the NICE risk stratification criteria as set out above. The second comparator result may be a pre-determined threshold indicative of the qSOFA criteria for risk of poor outcome as noted above.

The samples employed for a method of the invention will be whole blood samples in which case as indicated above superoxide production will strictly equate with leukocyte capacity for superoxide production (or alternatively stated ability to produce a respiratory burst). Such methodology importantly avoids centrifugation, which is known to affect cell reactivity, and also plating out of cells on glass slides which may also affect functionality. Blood samples as small as 10-20 µl (preferably 10 µl) will suffice obtained using a conventional finger lancing device.

In some instances, if need be or desired, the measured superoxide production above basal for each sample may be corrected by reference to the number of leukocytes or neutrophils in the sample. For example, it may be chosen to determine the challenge-induced increase of superoxide production above basal per 10⁹ neutrophils/I, designated the LIT-N score; see Example 3.

As already noted above, superoxide production is conveniently measured by known simple chemiluminescence measurement using luminol. Suitable protocols are disclosed for example in European Patent no. 1558929 of Oxford MediStress. An incubation temperature of 37- 37.5°C will be chosen and incubation continued for a pre-determined time of 10 minutes. As referred to above, the freeze-dried composition comprising PMA and luminol salt as supplied by Oxford MediStress for LCC testing enables suitable test results from a finger prick of whole blood to be attained in just 10 minutes and was used for the tests reported in Example 2 as a preferred reagent.

While a conventional luminometer may be employed for detection of the chemiluminescence, such light detectors require expensive and fragile photomultipler tubes. Hence, use of an alternative portable photon detector may be preferred. In particular, a silicon photomultipler (Si-PM) may, for example, be favoured. Such a photon detector is deemed more robust for the purpose and to combine greater cost-effectiveness with sufficient sensitivity of photon detection. A suitable hand-held luminometer is available again from Oxford MediStress as part of the CopingCapacity^{™} test kit, which also provides freeze-dried PMA/luminol-containing reagent composition as noted above.

A suitable photon detector may also be provided as a component of a mobile phone. Such a mobile luminometer for chemiluminescence detection has previously been proposed as part of a mobile chemistry platform (Roda et al. (2014) Anal. Chem. " Integrating Biochemiluminescence Detection on Smartphones; Mobile Chemistry Platform for Point-of-Need Analysis" ) and may be similarly employed to enable carrying out methods of the invention in locations even remote from any surgery or hospital, for example in a conflict zone where the likelihood of sepsis associated with injury is high.

It will be appreciated that methods of the invention have applicability for use with patients infected with a number of contagious pathogens such as bacteria, viruses, fungi or other agents known to induce sepsis in severe cases.

Whilst methods of the invention are of especial interest in diagnosing sepsis and monitoring sepsis progression in human patients or for providing a clinical indicator of sepsis progression or regression following treatment of such patients, it will be appreciated that such methods also have application to treatment in the veterinary field, especially in relation to other mammals.

Thus, the methodology of the invention can be envisaged as enabling all of the following in relation to management and treatment of sepsis and septic shock:
1. Leukocyte test as a predictor of sepsis and/or septic shock.
2. Leukocyte test as an objective marker of the effectiveness of therapies.
3. Use of a leukocyte test as an end point for therapies.
4. Leukocyte test for balancing and moderating therapeutic interventions (for example dosage levels and the time interval between dosages) optimising cell function (rather than just counting cells or looking at structure).
5. Leukocyte test as a controlled checkpoint during therapies.
6. Leukocyte test to optimise therapeutic intervention.
7. An early warning for recurrence

A method of the invention is:
- Simple, easy to use - results obtainable in 10 minutes, minimally invasive.
- Unit for protocol can be portable, not lab-based thereby minimising cost.
- Allows integration of treatments based on objective assessment of clinically relevant neutrophil functionality.
- Facilitates truly personalised objective patient care.

Of particular relevance to convenience of use as noted above is that there is no necessity to fractionate blood samples to obtain an isolated leukocyte or neutrophil fraction. Blood samples for carrying out a method of the invention are directly contacted with phorbol myristate acetate (PMA), more particularly for example the microbial product phorbol 12-myristate 13-acetate obtainable from Sigma-Aldrich. The chemical inducer may be conveniently stored in the form of a freeze-dried reagent composition, e.g. as a pellet, for dissolution in an appropriate buffer solution, e.g. phosphate-buffered saline.

For additional convenience coupled with high sensitivity, as indicated above, luminol may be conveniently supplied with the chemical inducer in a single reagent composition for addition to samples as exemplified by the commercially-available freeze-dried composition comprising PMA and luminol noted above.

In a further aspect of the invention, there is provided a system specifically configured to carry out a method of the invention as discussed above, the system comprising a photon detector such as a portable luminometer or Si-PM for quantitative detection of chemiluminescence and a system for analysing the results and configured to provide an alert for a neutrophil functionality level associated with risk of, or occurrence of, sepsis, or a particular sepsis status including septic shock. The same system may also be employed in monitoring sepsis or septic shock treatment as discussed above and provide additionally an alert favouring desirability of changing or stopping treatment.

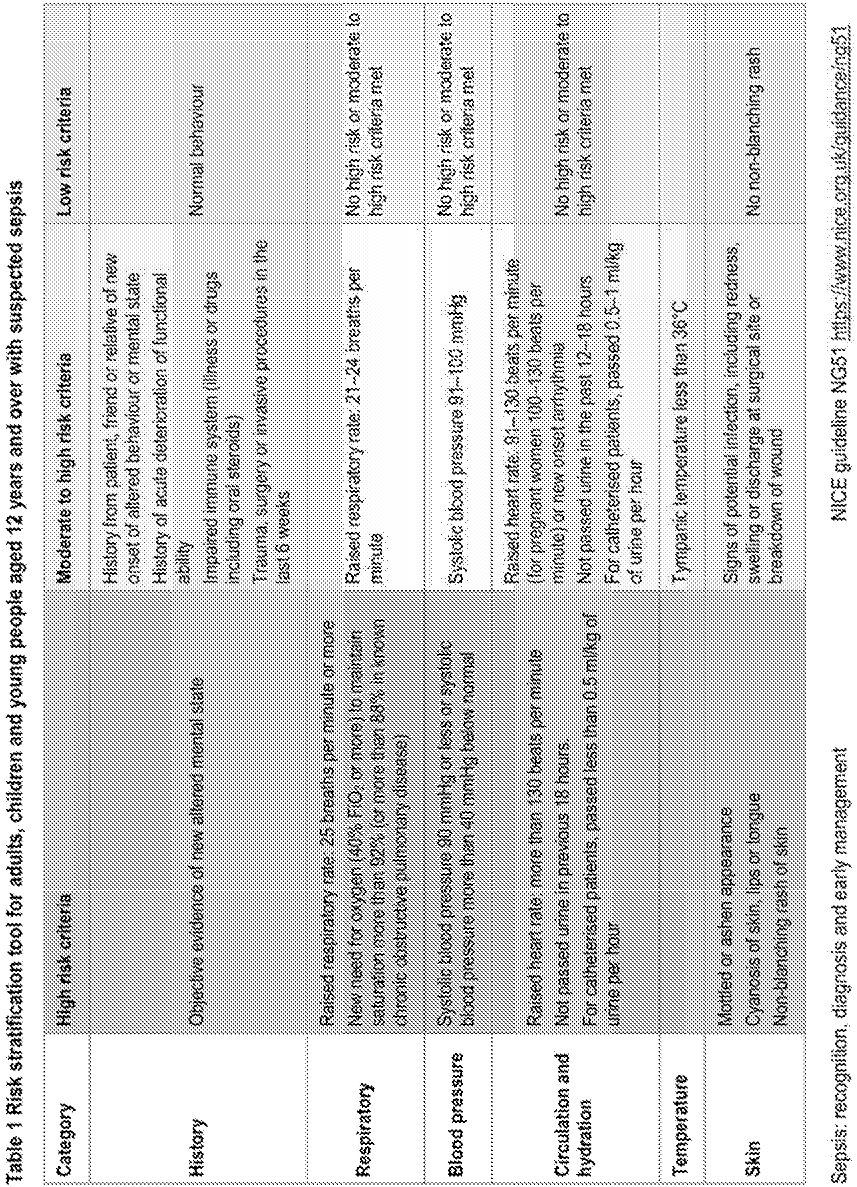

The following non-limiting examples illustrate the invention.

### Examples:

### Example 1: Example of protocol for use of PMA challenge to assess neutrophil functionality level in whole blood samples

To measure the background blood chemiluminescence level, 10 µl of whole blood is transferred into a silicon anti-reflective tube. 90 µl of 10⁻⁴ M luminol (5-amino-2,3-dihydro- 1,4 phthalazinedione; Sigma- Aldrich) diluted in phosphate buffer is added. The tube is then shaken gently. To measure chemiluminescence produced in response to PMA challenge, 20 µl of PMA (Sigma-Aldrich) at a concentration of 10⁻³ M is added. For each tube, chemiluminescence may be measured for 30 secs every 5 mins in a luminometer, for a total of 30 mins. When not in the luminometer, tubes are incubated at 37.5°C, e.g. in a dry block heater.

A single reading after incubation at 37.5 °C for 10 minutes may be found convenient and more preferable.

It will be appreciated that the same challenge test may be carried out with any sufficiently sensitive photon detector, e.g. a Si-PM may be employed

### Example 2: Comparison of LIT scores for patients with sepsis compared with healthy controls

The study was approved by a local ethics committee and conducted at an NHS teaching hospital in Birmingham, U.K. 12 patients in the local intensive care unit (ICU) suffering from sepsis and 20 healthy volunteers were recruited from hospital staff to the study. After consenting to the study, finger prick samples of blood (10 µl) were obtained from each patient in triplicate and analysed for neutrophil functionality level using freeze-dried PMA/ luminol reagent composition as commercially available from Oxford MediStress for LCC scoring. This involved mixing the blood with a buffered solution (100 µl) of reagents containing the freeze-dried PMA/luminol mixture. After incubation in a dry block heater for 10 minutes at 37.5 °C, the sample was evaluated for production of reactive oxygen species, measured using a portable luminometer (3M Clean-Trace^{®}). One set of samples (in triplicate) was obtained from each test subject.

The test results were analysed by grouping test subjects into two cohorts (sepsis or healthy volunteers). All the sepsis patients were scored as sepsis patients by reference to qSOFA scoring (respiratory rate ≥22/min, altered mentation and systolic blood pressure ≤100mm Hg). All the sepsis patients were also exhibiting hypotension requiring vasopressors and had serum lactate greater than 2mM.

LCC scores for neutrophil functionality level for each patient in these categories were averaged (mean LIT score) and these averages plotted as shown in Figure 1.

### Results:

- The data shows strong significant differences in LIT score between the two cohorts with the sepsis group having a greater than 10x average LIT score, compared with the healthy volunteers cohort.
- The average (mean ± SD) LIT score for the sepsis group was 2526 ± 1616 while the mean ± SD LIT score for the "healthy volunteers" cohort was_167±99_.
- Statistically significant difference (via t-test) in LIT scores was shown between the two cohorts.

### Mean (SD) and median (IQR) values for sepsis and healthy groups

| Group | N | Mean ± SD | Median (IQR) |
|---|---|---|---|
| healthy | 18 | 167 ± 99 | 153 (112) |
| sepsis | 12 | 2526 ± 1616 | 2120 (2024) |

These results for the first time demonstrated that neutrophil functionality level as assessed by LIT score measured using the Oxford MediStress system can be used as an indicative measure of the onset of sepsis, especially, for example, high risk sepsis as may warrant close observation and treatment in an ICU, and as a tool to monitor sepsis progression, interventions and recovery.

### Example 3: Use of LIT score relative to neutrophil count

LIT score was also plotted against neutrophil count in the blood samples of the sepsis patient cohort with the neutrophil count being expressed as neutrophils x 10⁹/l. The gradient of this line was taken to determine the LIT-N value which represented the increase in LIT for each increase of 1 x 10⁹/l in the blood neutrophil count. The median LIT-N value for sepsis patients was 115.

LIT-N score was also looked at in relation to 28- day mortality of patients on the ICU. The LIT- N score for a patient with non-covid sepsis who died in the relevant period was markedly elevated prior to death at 419.

### References:

Novosad SA, Sapiano MRP, Grigg C, et al. Vital signs: epidemiology of sepsis: prevalence of health care factors and opportunities for prevention. Morb Mortal Wkly Rep. 2016;65(33):864-869. doi: 10.15585/mmwr.mm6533e1.
Vincent J-L, Rello J, Marshall J, et al. International study of the prevalence and outcomes of infection in intensive care units. JAMA. 2009;302(21) ,2323-2329.
Gyawali B, Ramakrishna K, Dhamoon AS. Sepsis: The evolution in definition, pathophysiology, and management. SAGE Open Med. 2019 Mar 21;7:2050312119835043. doi: 10.1177/2050312119835043. PMID: 30915218; PMCID: PMC6429642.
Hu et al. Cell Signal (1999) 11, 335-360
Kaukonen K, Bailey M, Suzuki S, et al. Mortality related to severe sepsis and septic shock among critically III patients in Australia and New Zealand, 2000-2012. JAMA 2014; 311(13): 1308-1316.
Kumar A, Roberts D, Wood KE, et al. Duration of hypotension before initiation of effective antimicrobial therapy is the critical determinant of survival in human septic shock. Crit Care Med. 2006; 34(6): 1589-1596.
Roda et al. Integrating Bioluminescence Detection on Smartphones: Mobile Chemistry Platform for Point-of-Need Analysis. Anal. Chem. (2014) 86(15), 7299-7304
Segal AW. How neutrophils kill microbes. Ann. Rev. Immunol. 2005;23:197-223.
Singer et al. (2016) The Third International Consensus Definitions for Sepsis and Septic Shock (Sepsis-3) JAMA 315, 801-810.

## Claims

1. A method of providing an indicative diagnosis of the onset or occurrence of sepsis or septic shock, or determining the onset or occurrence of sepsis or septic shock, in a subject known to have an infection with an agent capable of giving rise to sepsis or suspected of having such an infection, which comprises:
(a) directly contacting a test whole blood sample of 10-20µl obtained from said subject with an inducer capable of stimulating superoxide production in neutrophils, said inducer being phorbol myristate acetate (PMA) and said contacting being at a temperature of 37-37.5°C under conditions suitable for such stimulation;
(b) determining the increase of superoxide production above basal in said test sample after a pre-determined time period of 10 minutes to obtain a first test result, wherein superoxide production is detected by chemiluminescence detection using luminol and
(c) comparing said first test result with a second comparator result which is a threshold derived by applying steps (a) and (b) to whole blood samples of healthy individuals and utilising the increase of chemiluminescence of said first test result above the increase of chemiluminescence of said second comparator result;
whereby the onset, presence or absence of sepsis or septic shock in said subject is indicated or determined.

2. A method as claimed in claim 1 for determining the onset or occurrence of high risk sepsis or septic shock, wherein high risk sepsis accords with the criteria of the UK NICE guidelines for such risk stratification, qSOFA score for such sepsis status according to The International Sepsis Task Force or alternative criteria for equivalent assignment of sepsis status.

3. A method of ranking or scoring a sepsis patient on the basis of sepsis progression or presence of septic shock, which comprises:
(a) directly contacting a test whole blood sample of 10-20µl obtained from said patient with an inducer capable of stimulating superoxide production in neutrophils, said inducer being phorbol myristate acetate (PMA) and said contacting being at a temperature of 37-37.5°C under conditions suitable for such stimulation;
(b) determining the increase of superoxide production above basal in said test sample after a pre-determined time period of 10 minutes to obtain a first test result, wherein superoxide production is detected by chemiluminescence detection using luminol and
(c) comparing said first test result with one or more second comparator results each of which is a pre-determined threshold which correlates with one or more criteria for sepsis progression or septic shock so as to rank or score sepsis progression or presence of septic shock in the subject,
wherein the one or more second comparator results are derived by applying steps (a) and (b) to whole blood samples of healthy individuals and utilising the increase of chemiluminescence of said first test result above the increase of chemiluminescence of said second comparator result.

4. A method as claimed in claim 3, wherein said first test result is compared with a pre-determined threshold for onset or occurrence of high risk sepsis or septic shock.

5. A method for determining the effectiveness of the treatment of sepsis or septic shock in a subject, said method comprising, following or during sepsis or septic shock treatment,
(a) directly contacting a test whole blood sample of 10-20µl obtained from said subject with an inducer capable of stimulating superoxide production in neutrophils, said inducer being phorbol myristate acetate (PMA) and said contacting being at a temperature of 37-37.5°C under conditions suitable for such stimulation;
(b) determining the increase of superoxide production above basal in said test sample after a pre-determined time period of 10 minutes to obtain a first test result, wherein superoxide production is detected by chemiluminescence detection using luminol and
(c) comparing said first test result with a second comparator result which is a threshold derived by applying steps (a) and (b) to whole blood samples of healthy individuals and utilising the increase of chemiluminescence of said first test result above the increase of chemiluminescence of said second comparator result; and
wherein the increase in chemiluminescence above basal in said test sample is compared with the increase in chemiluminescence above basal in a second comparator sample which has been taken from the subject at an earlier time point at the start or during treatment,
whereby reduction in induced chemiluminescence in the test sample compared with in said second comparator sample is indicative of depressive effect of the treatment on neutrophil functionality.

6. A method as claimed in any one of claims 1 to 5 wherein in step (b) the increase of superoxide production above basal is determined per 10⁹ neutrophils/I to obtain a LIT- N score.

7. A system specifically configured for carrying out a method according to any one of claims 1 to 6, said system comprising a photon detector for quantitative detection of chemiluminescence and a system for analysing the results and configured to provide an alert for a neutrophil functionality level associated with risk, or occurrence of, sepsis or a particular sepsis status including septic shock.

8. A system as claimed in claim 7, wherein said photon detector is a portable luminometer or provided as a component of a mobile phone.

## Patentansprüche

1. Verfahren zum Bereitstellen einer indikativen Diagnose des Auftretens oder Vorkommens von Sepsis oder septischem Schock oder Bestimmen des Auftretens oder Vorkommens von Sepsis oder septischem Schock bei einem Subjekt, von dem bekannt ist, dass es eine Infektion mit einem Mittel aufweist, das in der Lage ist, Sepsis hervorzurufen, oder von dem vermutet wird, dass es eine solche Infektion aufweist, das Folgendes umfasst:
(a) direktes Kontaktieren einer Testvollblutprobe von 10-20 µl, die von dem Subjekt erhalten wird, mit einem Induktor, der in der Lage ist, Superoxidproduktion in Neutrophilen zu stimulieren, wobei der Induktor Phorbolmyristatacetat (PMA) ist und das Kontaktieren bei einer Temperatur von 37-37,5 °C unter Bedingungen ist, die für solche Stimulation geeignet sind;
(b) Bestimmen des Anstiegs von Superoxidproduktion über basal in der Testprobe nach einem vorbestimmten Zeitraum von 10 Minuten, um ein erstes Testergebnis zu erhalten, wobei Superoxidproduktion durch Chemilumineszenzdetektion unter Verwendung von Luminol detektiert wird, und
(c) Vergleichen des ersten Testergebnisses mit einem zweiten Vergleichsergebnis, das ein Schwellenwert ist, der durch Anwenden von Schritt (a) und (b) auf Vollblutproben von gesunden Individuen und Nutzen des Anstiegs von Chemilumineszenz des ersten Testergebnisses über den Anstieg von Chemilumineszenz des zweiten Vergleichsergebnisses abgeleitet wird;
wodurch das Auftreten, Vorhandensein oder Nichtvorhandensein von Sepsis oder septischem Schock bei dem Subjekt angegeben oder bestimmt wird.

2. Verfahren nach Anspruch 1 zum Bestimmen des Auftretens oder Vorkommens von Sepsis mit hohem Risiko oder septischem Schock, wobei Sepsis mit hohem Risiko den Kriterien der britischen NICE-Richtlinien für solche Risikostratifizierung, der qSOFA-Bewertung für solchen Sepsisstatus gemäß der International Sepsis Task Force oder alternativen Kriterien für äquivalente Zuordnung des Sepsisstatus entspricht.

3. Verfahren zum Einstufen oder Bewerten eines Sepsispatienten auf der Grundlage von Sepsisprogression oder Vorhandensein von septischem Schock, das Folgendes umfasst:
(a) direktes Kontaktieren einer Testvollblutprobe von 10-20 µl, die von dem Patient erhalten wird, mit einem Induktor, der in der Lage ist, Superoxidproduktion in Neutrophilen zu stimulieren, wobei der Induktor Phorbolmyristatacetat (PMA) ist und das Kontaktieren bei einer Temperatur von 37-37,5 °C unter Bedingungen ist, die für solche Stimulation geeignet sind;
(b) Bestimmen des Anstiegs von Superoxidproduktion über basal in der Testprobe nach einem vorbestimmten Zeitraum von 10 Minuten, um ein erstes Testergebnis zu erhalten, wobei Superoxidproduktion durch Chemilumineszenzdetektion unter Verwendung von Luminol detektiert wird, und
(c) Vergleichen des ersten Testergebnisses mit einem oder mehreren zweiten Vergleichsergebnissen, von denen jedes ein vorbestimmter Schwellenwert ist, der mit einem oder mehreren Kriterien für Sepsisprogression oder septischen Schock korreliert, um Sepsisprogression oder Vorhandensein von septischem Schock bei dem Subjekt einzustufen oder zu bewerten,
wobei das eine oder die mehreren zweiten Vergleichsergebnisse durch Anwenden von Schritt (a) und (b) auf Vollblutproben von gesunden Individuen und Nutzen des Anstiegs von Chemilumineszenz des ersten Testergebnisses über den Anstieg von Chemilumineszenz des zweiten Vergleichsergebnisses abgeleitet werden.

4. Verfahren nach Anspruch 3, wobei das erste Testergebnis mit einem vorbestimmten Schwellenwert für Auftreten oder Vorkommen von Sepsis mit hohem Risiko oder septischem Schock verglichen wird.

5. Verfahren zum Bestimmen der Wirksamkeit der Behandlung von Sepsis oder septischem Schock bei einem Subjekt, wobei das Verfahren nach oder während Behandlung von Sepsis oder septischem Schock Folgendes umfasst
(a) direktes Kontaktieren einer Testvollblutprobe von 10-20 µl, die von dem Subjekt erhalten wird, mit einem Induktor, der in der Lage ist, Superoxidproduktion in Neutrophilen zu stimulieren, wobei der Induktor Phorbolmyristatacetat (PMA) ist und das Kontaktieren bei einer Temperatur von 37-37,5 °C unter Bedingungen ist, die für solche Stimulation geeignet sind;
(b) Bestimmen des Anstiegs von Superoxidproduktion über basal in der Testprobe nach einem vorbestimmten Zeitraum von 10 Minuten, um ein erstes Testergebnis zu erhalten, wobei Superoxidproduktion durch Chemilumineszenzdetektion unter Verwendung von Luminol detektiert wird, und
(c) Vergleichen des ersten Testergebnisses mit einem zweiten Vergleichsergebnis, das ein Schwellenwert ist, der durch Anwenden von Schritt (a) und (b) auf Vollblutproben von gesunden Individuen und Nutzen des Anstiegs von Chemilumineszenz des ersten Testergebnisses über den Anstieg von Chemilumineszenz des zweiten Vergleichsergebnisses abgeleitet wird; und
wobei der Anstieg der Chemilumineszenz über basal in der Testprobe mit dem Anstieg der Chemilumineszenz über basal in einer zweiten Vergleichsprobe verglichen wird, die dem Subjekt zu einem früheren Zeitpunkt zu Beginn oder während Behandlung entnommen worden ist,
wodurch Reduzierung der induzierten Chemilumineszenz in der Testprobe verglichen mit der zweiten Vergleichsprobe depressive Wirkung der Behandlung auf Neutrophilenfunktionalität anzeigt.

6. Verfahren nach einem der Ansprüche 1 bis 5, wobei in Schritt (b) der Anstieg von Superoxidproduktion über basal pro 10⁹ Neutrophilen/l bestimmt wird, um eine LIT-N-Bewertung zu erhalten.

7. System, das spezifisch konfiguriert ist, um ein Verfahren gemäß einem der Ansprüche 1 bis 6 durchzuführen, wobei das System einen Photonendetektor zur quantitativen Detektion von Chemilumineszenz und ein System zum Analysieren der Ergebnisse umfasst und konfiguriert ist, um eine Warnung für ein Neutrophilenfunktionalitätsniveau assoziiert mit Risiko oder Vorkommen von Sepsis oder einem bestimmten Sepsisstatus beinhaltend septischen Schock bereitzustellen.

8. System nach Anspruch 7, wobei der Photonendetektor ein tragbares Luminometer ist oder als Komponente eines Mobiltelefons bereitgestellt ist.

## Revendications

1. Procédé de fourniture d'un diagnostic indicatif de l'apparition ou la survenue d'une septicémie ou d'un choc septique, ou de détermination de l'apparition ou la survenue d'une septicémie ou d'un choc septique, chez un sujet connu pour présenter une infection par un agent capable de provoquer une septicémie ou suspecté de présenter une telle infection, qui comprend :
(a) la mise en contact direct d'un échantillon de sang total de test de 10 à 20 µl obtenu auprès dudit sujet avec un inducteur capable de stimuler la production de superoxyde chez les neutrophiles, ledit inducteur étant l'acétate de myristate de phorbol (PMA) et ladite mise en contact étant à une température de 37 à 37,5° C dans des conditions appropriées pour une telle stimulation ;
(b) la détermination de l'augmentation de la production de superoxyde au-dessus de la valeur basale dans ledit échantillon de test après une période de temps prédéterminée de 10 minutes pour obtenir un premier résultat de test, dans lequel la production de superoxyde est détectée par détection par chimiluminescence à l'aide de luminol et
(c) la comparaison dudit premier résultat de test à un second résultat de comparateur qui est un seuil dérivé en appliquant les étapes (a) et (b) à des échantillons de sang total d'individus sains et en utilisant l'augmentation de la chimioluminescence dudit premier résultat de test au-dessus de l'augmentation de la chimioluminescence dudit second résultat de comparateur ;
moyennant quoi l'apparition, la présence ou l'absence d'une septicémie ou d'un choc septique chez ledit sujet est indiquée ou déterminée.

2. Procédé selon la revendication 1 permettant la détermination de l'apparition ou la survenue d'une septicémie à haut risque ou d'un choc septique, dans lequel la septicémie à haut risque correspond aux critères des lignes directives du NICE britannique pour une telle stratification du risque, au score qSOFA pour un tel statut de septicémie selon le Groupe de Travail International sur la Septicémie ou d'autres critères permettant une attribution équivalente du statut de septicémie.

3. Procédé de classement ou de notation d'un patient atteint de septicémie sur la base de la progression de la septicémie ou de la présence d'un choc septique, qui comprend :
(a) la mise en contact direct d'un échantillon de sang total de test de 10 à 20µl obtenu auprès dudit sujet avec un inducteur capable de stimuler la production de superoxyde chez les neutrophiles, ledit inducteur étant l'acétate de myristate de phorbol (PMA) et ladite mise en contact étant à une température de 37 à 37,5° C dans des conditions appropriées pour une telle stimulation ;
(b) la détermination de l'augmentation de la production de superoxyde au-dessus de la valeur basale dans ledit échantillon de test après une période de temps prédéterminée de 10 minutes pour obtenir un premier résultat de test, dans lequel la production de superoxyde est détectée par détection par chimiluminescence à l'aide de luminol et
(c) la comparaison dudit premier résultat de test à un ou plusieurs seconds résultats de comparateur dont chacun est un seuil prédéterminé qui est en corrélation avec un ou plusieurs critères pour la progression de la septicémie ou le choc septique de manière à classer ou à noter la progression de la septicémie ou la présence d'un choc septique chez le sujet,
dans lequel l'un ou plusieurs seconds résultats de comparateur sont dérivés en appliquant les étapes (a) et (b) à des échantillons de sang total d'individus sains et en utilisant l'augmentation de la chimioluminescence dudit premier résultat de test au-dessus de l'augmentation de la chimioluminescence dudit second résultat de comparateur.

4. Procédé selon la revendication 3, dans lequel le premier résultat de test est comparé à un seuil prédéterminé d'apparition ou de survenue d'une septicémie ou d'un choc septique à haut risque.

5. Procédé permettant la détermination de l'efficacité du traitement d'une septicémie ou d'un choc septique chez un sujet, ledit procédé comprenant, après ou pendant un traitement de septicémie ou de choc septique,
(a) la mise en contact direct d'un échantillon de sang total de test de 10 à 20µl obtenu auprès dudit sujet avec un inducteur capable de stimuler la production de superoxyde chez les neutrophiles, ledit inducteur étant l'acétate de myristate de phorbol (PMA) et ladite mise en contact étant à une température de 37 à 37,5° C dans des conditions appropriées pour une telle stimulation ;
(b) la détermination de l'augmentation de la production de superoxyde au-dessus de la valeur basale dans ledit échantillon de test après une période de temps prédéterminée de 10 minutes pour obtenir un premier résultat de test, dans lequel la production de superoxyde est détectée par détection par chimiluminescence à l'aide de luminol et
(c) la comparaison dudit premier résultat de test à un second résultat de comparateur qui est un seuil dérivé en appliquant les étapes (a) et (b) à des échantillons de sang total d'individus sains et en utilisant l'augmentation de la chimioluminescence dudit premier résultat de test au-dessus de l'augmentation de la chimioluminescence dudit second résultat de comparateur ; et
dans lequel l'augmentation de la chimiluminescence au-dessus de la valeur basale dans ledit échantillon de test est comparée à l'augmentation de la chimiluminescence au-dessus de la valeur basale dans un second échantillon de comparateur qui a été prélevé sur le sujet à un moment antérieur au début ou pendant le traitement,
moyennant quoi la réduction de la chimiluminescence induite dans l'échantillon de test par rapport audit second échantillon de comparateur indique un effet dépressif du traitement sur la fonctionnalité des neutrophiles.

6. Procédé selon l'une quelconque des revendications 1 à 5, dans lequel à l'étape (b), l'augmentation de la production de superoxyde au-dessus de la valeur basale est déterminée pour 10⁹ neutrophiles/l pour obtenir un score LIT-N.

7. Système spécifiquement configuré pour effectuer un procédé selon l'une quelconque des revendications 1 à 6, ledit système comprenant un détecteur de photons pour la détection quantitative de la chimioluminescence et un système pour analyser les résultats et configuré pour fournir une alerte pour un niveau de fonctionnalité des neutrophiles associé à un risque ou à la survenue d'une septicémie ou d'un état septique particulier, y compris un choc septique.

8. Système selon la revendication 7, dans lequel le détecteur de photons est un luminomètre portable ou fourni en tant que composant d'un téléphone mobile.
